# EUROPEAN PATENT SPECIFICATION

(11) **EP 1 487 409 B1**
(45) Date of publication and mention of the grant of the patent: **22.04.2009**
(21) Application number: 03716645.1
(22) Date of filing: 17.03.2003
(51) Int. Cl.: A61K 9/08, A61K 9/10, A61K 9/72

(54) **HIGHLY AQUEOUS LIQUID CARRIER FORMULATIONS**
FLÜSSIGE TRÄGERFORMULIERUNGEN MIT HOHEM WASSERGEHALT
FORMULATIONS DE SUPPORT LIQUIDES A TENEUR EN EAU ELEVEE

(30) Priority: 22.03.2002 US 104662
(43) Date of publication of application: 22.12.2004
(73) Proprietor: BATTELLE MEMORIAL INSTITUTE, Columbus, OH 43201-2693 (US)
(72) Inventor: COWAN, Siu, Man, L., Lewis Center, OH 43035 (US); PALMER, Donna, T., San Diego, CA 92131 (US)
(74) Representative: Turi, Michael
(86) International application number: PCT/US2003/008157
(87) International publication number: WO 2003/082242

(56) References cited:
- EP-A- 0 444 778
- WO-A-01/74431
- WO-A-02/43750
- WO-A-96/19197
- US-A- 5 215 739
- US-B1- 6 339 107

## Description

### FIELD OF THE INVENTION

The present invention relates to the field of pulmonary drug delivery of highly aqueous liquid aerosol solutions and suspensions of medicaments using an electrohydrodynamic spray/aerosolization means. Specifically, the present invention provides highly aqueous liquid carrier formulations and methods for the aerosol delivery by inhalation of highly aqueous liquid compositions to the respiratory tract using electrohydrodynamic means.

### BACKGROUND OF THE INVENTION

Drugs for treating respiratory and nasal disorders are frequently administered in aerosol formulations through the mouth or nose. One widely used method for dispensing such aerosol drug formulations involves making a suspension formulation of the drug as a finely divided powder in a liquefied gas known as a propellant. The suspension is dispersed by activation of a dose-metering valve affixed to the container. Systems for dispensing drugs in this way are known as "metered dose inhalers" (MDI's). See Peter Byron, Respiratory Drug Delivery, CRC Press, Boca Raton, Fla. (1990) for a general background on this form of therapy.

There are inherent problems associated with the delivery of a drug as a powder. Powders have a tendency to adhere to the inner surfaces, i.e., walls of the can, valves, and caps, of the MDI, which can lead to the patient getting significantly less than the prescribed amount of drug upon each activation of the MDI. The problem is particularly acute with hydrofluoroalkane (fluorocarbon) propellant systems, e.g., P134a and P227, under development in recent years to replace chlorofluorocarbons such as P11, P114, and P12. Further, halohydrocarbon propellants such as chlorofluorocarbons and to a lesser extent the hydroflurocarbons have adverse environmental effects.

Nebulizers offer an alternative method of administering therapeutic agents to the lungs. These devices work by means of an air jet or an ultrasonic pulse that is applied to a solution producing a fine mist through a critical orifice. Therapeutic agents dissolved or suspended in the solution can be incorporated into the mist. The patient then breathes the mist in and out over the course of several minutes of treatment, during which 1 to 3 ml of the drug formulation is typically nebulized. There is no need for coordination between hand action and breathing, making the nebulizer easier to use for patients. It may be possible, in some cases, to administer drugs not soluble in aqueous solution by nebulizing them in suspension. However, the droplet size of nebulized drug-containing suspensions cannot be smaller than that of the suspended particles. Therefore, the finer droplets produced from these systems would not contain any drug.

Nebulizer devices and methodology can be quite useful when the precise dosing of the drug being delivered to the patient is not of particular importance. In some situations, the nebulizer creates a mist from an aqueous solution containing a drug, e.g., a bronchodilator that can be inhaled by the patient until the patient feels some improvement in lung function. When precise dosing is more important, the nebulizer device and delivery methodology suffers from many of the disadvantages of metered dose inhaler devices as described above. In addition, nebulizers are generally large in size and are not easily transportable devices like MDIs.

Generally, a nebulizer can only be used within a fixed location such as the patient's home, the doctors office and/or hospital. However, portable nebulizers are known, such as that taught in PCT application WO92/11050. Drug formulations placed in nebulizers are generally diluted prior to delivery. The entire diluted formulation must generally be administered at a single dosing event in order to maintain the desired level of sterility and the nebulizer must be cleaned after use. Yet another disadvantage of nebulizers is that they produce an aerosol, which has a wide distribution of particle sizes not all of which are of an appropriate size to reach the targeted areas of the lung.

Dispensing devices are known which produce a finely divided spray of liquid droplets by electrostatic means, more properly referred to as electrohydrodynamic ("EHD") means. Electrohydrodynamic sprayers have found use in many areas of industry, in medicine for the administration of medicaments by inhalation, in agriculture for crop spraying, and in the automobile industry for paint spraying.

In a typical EHD device, a fluid delivery means delivers fluid to be aerosolized to a nozzle maintained at high electric potential. One type of nozzle used in EHD devices is a capillary tube that is capable of conducting electricity. An electric potential is placed on the capillary tube which charges the fluid contents such that, as the fluid emerges from the tip or end of the capillary tube, a so-called Taylor cone is formed. This cone shape results from a balance of the forces of electric charge on the fluid and the fluid's own surface tension. Desirably, the charge on the fluid overcomes the surface tension and at the tip of the Taylor cone, a thin jet of fluid forms and subsequently and rapidly separates a short distance beyond the tip into an aerosol. Studies have shown that this aerosol (often described as a soft cloud) has a uniform droplet size and a high velocity leaving the tip but that it quickly decelerates to a very low velocity a short distance beyond the tip.

EHD sprayers produce charged droplets at the tip of the nozzle. Depending on the use, these charged droplets can be partially or fully neutralized (with a reference or discharge electrode in the sprayer device) or not. When the EHD device is used to deliver therapeutic aerosols, it is preferred that the aerosol be completely electrically neutralized prior to inhalation by the user to permit the aerosol to reach the pulmonary areas where the particular therapeutic formulation is most effective. However, if nasal deposition of the aerosol is desired, an EHD sprayer without means for discharging or means for partially discharging an aerosol might be preferred since the aerosol would have a residual electric charge as it leaves the sprayer so that the droplets would be attracted to and tightly adhere to the surface of the nares.

Various EHD devices are known in the art, for example, US Pat. No. 6,302,331, US Patent 6,105,877 and WO 99/07478. Although, the various patents disclose different methods for obtaining therapeutic aerosols having an aerosol droplet size of in the range of from 0.1 um to 25 um, very little direction is provided regarding suitable carrier liquids for use in the pulmonary administration of therapeutic agents as solutions or suspensions using EHD spraying/aerosolization devices.

Various liquid carrier vehicles are described in the art for use with preparing formulations of medicaments to be administered via inhalation. Ethanol/propylene glycol liquid carrier vehicles are described in US Pat. No. 6,105,571 and in WO 99/07478. However, the prior art does not teach how to aerosolize highly aqueous carrier liquids such as those described and claimed herein. Highly aqueous carrier liquids are desirable because water is the safest liquid component of any base formulation for use with EHD. Further, water in comparison to halogenated propellant gasses is economical and safe for the environment.

US Pat. No. 5,660,166 describes a system for delivering an aerosol that is a liquid, flowable formulation consisting essentially of a pharmaceutically active drug dissolved or dispersed in an ethanol or ethanol/water carrier liquid. The reference specifically teaches that the surface tension of the liquid should be low. Despite the desirability of using highly aqueous carrier liquids for aerosol formulations of medicaments sprayed and aerosolized using EHD devices, such highly aqueous carrier liquids have heretofore not been used because neat water does not spray by EHD means under practical operating conditions due to the high surface tension of water (72 dynes/cm). For example, US Pat. No. 4,829,996 teaches that, predominantly aqueous formulations are not completely satisfactory, since water has a high surface tension making it difficult to spray.

While it is possible to spray and aerosolize water using an EHD device, it is necessary to use very high voltages and slow flow rates. Such conditions are not practical conditions under which to administer a medicament aerosol to a patient. The high voltage is a safety hazard and the slow flow rate significantly extends the time of treatment from minutes to hours. Surprisingly, as described herein, the highly aqueous liquid carrier vehicle of the invention may be aerosolized at significant flow rates using reasonable voltages.

US Pat. No. 5,873,523 (Gomez *et. al.*) at Col. 4, lines 53-60, teaches that because of the large surface tension of water, the establishment of stable sprays in air is generally presented by the occurrence of internal electric breakdown in the gaseous environment surrounding the spray and its destabilizing consequences on the spray behavior. Gomez overcomes this problem and is able to spray aqueous solutions by producing aerosols using CO₂. When air is replaced by CO₂ during the formation of the Taylor cone, droplets of nearly monodisperse size distributions in the 2 - 8 µm diameter range were produced in pure water and in hypotonic saline at flow rates ranging from 7- 20 µl/min.

US 6,339,107 discloses a formulation for delivering 13-cis-retinoic acid via an EHD device which uses Cremophor RH40 as an emulsifier.

Surprisingly, the highly aqueous canier-liquids of the present invention produce stable sprays in air when sprayed using an EHD device without the necessity of using CO₂ in the formation of the aerosol. Accordingly, it is an object of this invention to provide a highly aqueous liquid carrier vehicle in which a pharmaceutically active agent may be dissolved or suspended and the resulting solution or suspension delivered to a patient in need of treatment using an EHD device via pulmonary administration and where such delivery is made at a high flow rate unlike the slow flow rates of the prior art.

Another object of the invention is to provide compositions of pharmaceutically active agents which are administered to the pulmonary tract of a patient via inhalation of an aerosol where the aerosol is produced using an EHD device. Yet, another embodiment of the invention is directed to a method for delivering an effective amount of a pharmaceutically active agent to the respiratory tract of a patient in need of treatment.

### BRIEF DESCRIPTION OF DRAWINGS

FIG. 1 is a plot showing the particle size distribution of the aerosol sprayed using EHD means of a 30% EtOH/70% H₂O liquid carrier contained 0.5% C10-glucose surfactant and 10% propylene glycol.
FIG. 2 is a plot showing the particle size distribution of the aerosol sprayed using an EHD means of a 30% EtOH/70% H₂O liquid carrier containing 0.5% C10-glucose surfactant, 0.1% Vitamin E TPGS and 10% propylene glycol.
FIG. 3 is a plot of the particle size distribution of an aerosol produced from a liquid carrier consisting of 30% EtOH/70% H₂O.
FIG. 4 is a plot of the particle size distribution of an aerosol produced from a liquid carrier consisting o 30% EtOH/70% H₂O, plus 10% propylene glycol.

### SUMMARY OF THE INVENTION

The invention is directed to a highly aqueous liquid carrier for a pharmaceutically active agent administered to a patient via inhalation of an aerosol wherein said aerosol is produced by an electrohydrodynamic spraying device according to claim 1.

The dependent claims are useful embodiments of the invention.

A use of the liquid carrier vehicle is also claimed in claim 14.

### DETAILED DESCRIPTION OF THE INVENTION

The invention is directed to a liquid carrier vehicle for a pharmaceutical active agent administered to a patient via inhalation of an aerosol wherein said aerosol is produced by an electrohydrodynamic spraying according to claim 1.

The highly aqueous liquids carrier vehicles of the invention are useful for preparing aerosols for the delivery of "pharmaceutically active agents" to the "respiratory tract" of a patient using an EHD spraying/aerosolization device. The term "respiratory tract" as used herein includes the upper airways, including the oropharynx and larynx, followed by the lower airways, which include the trachea followed by bifurcations into the bronchi and bronchioli. The upper and lower airways are called the conductive airways. The terminal bronchioli then divide into respiratory bronchioli, which then lead to the ultimate respiratory zone, the alveoli, or deep lung. Gonda, I. "Aerosols for delivery of therapeutic and diagnostic agents to the respiratory tract," in Critical Reviews in Therapeutic Drug Carrier Systems, 6:273-313, (1990). Usually, the deep lung, or alveoli, is the primary target of inhaled therapeutic aerosols for systemic delivery. As used herein, the term "respiratory tract" is additionally meant to include administration of the highly aqueous liquid formulations buccally and to the nasal passages and to the mucosa of the bucca.

The term "liquid carrier" as used herein refers to the liquid vehicle in which the drug to be administered is dissolved or suspended. The liquid carrier is "highly aqueous", i.e., it is required to contain at least about 50% water V/V and preferably about 76% V/V water in addition to no more than about 40% V/V ethanol, no more than about 30% V/V of a co-solvent, one or more "pharmaceutically acceptable excipients" and one or more "derivatized carbohydrate surfactants".

The highly aqueous carrier liquid formulations of the invention includes minor amounts, that is, from about 0.5% to about 10% W/V and preferably from about 0.5% to from about 5% W/V of a "pharmaceutically acceptable excipient". Pharmaceutically acceptable excipients are those recognized by the FDA as being safe for use in humans. Additives selected from antioxidants, e.g., Vitamin E, Vitamin E TPGS (α - alpha tocopferol polyethylene glycol 1000 succinate), ascorbic acid, anti-microbials; e.g, parabens, pH adjusting agents, e.g., sodium hydroxide and hydrochloric acid, tonicity adjusting agents, e.g., sodium chloride and viscosity adjusting agents, e.g., polyvinyl pyrrolidone are used herein.

While the selection of any particular pharmaceutically acceptable excipient is within the skill of the art, the decision which one, will be made taking into account the purpose of the excipient in as specific liquid carrier vehicle. In order to be pharmaceutically acceptable any formulation excipient used in the carrier liquids of the invention should be recognized by the FDA as safe for use in humans. Additionally, an excipient should have no effect or minimal effect on the sprayability of formulations of a drug dissolved or suspended in a liquid carrier using an EHD spraying means.

From about 0% V/V to about 30% V/V of a co-solvent may be used in the liquid carrier vehicle of the invention and preferably from about 2.5% to about 10% V/V will be used and more preferably about 5% V/V. The term "co-solvent" refers to mono- and polyvalent alcohols such as propylene glycol, glycerol, and polyethylene glycol (PEG) having an average molecular weight between about 200 and 4000, preferably between about 200 and 400.

As used herein, the term "pharmaceutically active agent" refers to biologically active agents that are used for diagnostic purposes as well as agents that are administered to human or animal patients as the active drug substance for treatment of a disease or condition. Such active drug substances are administered to a patient in a "pharmaceutically effective amount" to treat a disease or condition. As would be recognized by one skilled in the art, by "effective amount" is meant an amount of a pharmaceutically active agent having a therapeutically relevant effect on the disease or condition to be treated. A therapeutically relevant effect relieves to some extent one or more symptoms of the disease or condition in a patient or returns to normal either partially or completely one or more physiological or biochemical parameters associated with or causative of the disease or condition. Specific details of the dosage of a particular active drug may be found in its labeling, i.e., the package insert (see 21 CFR § 201.56 & 201.57) approved by the United States Food and Drug Administration.

When a pharmaceutically active agent is added to the liquid carrier a solution is produced if the drug is soluble in the liquid carrier and a suspension is produced if the drug is insoluble. The term "suspension" as used herein is given its ordinary meaning and refers to particles of drug or aggregates of particles of drug suspended in the liquid carrier. When the drug is present as a suspension the particles of drug will likely be in the nanometer range.

The unique derivatized carbohydrate surfactants useful in the present invention should have low animal toxicity and immunogenicity. The derivatized carbohydrate surfactants are highly effective in lowering surface tension of the highly aqueous liquid carrier vehicle as it is discharged from the EHD spraying means. Further, the derivatized carbohydrate surfactants described herein many be used at low concentrations. In general, from about 0.05% to about 10% W/V of the carrier liquid and preferably from about 0.3% to about 5% W/V of the liquid carrier vehicle may be used in the liquid carrier vehicles of the Invention.

The choice of a particular derivatized carbohydrate surfactant for use in a particular liquid carrier vehicle will be made considering the physical and chemical properties of the drug to be aerosolized, e.g. is the drug soluble in water or very insoluble, the amount of ethanol in the liquid carrier vehicle, the nature and amount of any co-solvent or excipient in the liquid carrier vehicle, the desired particle size of the resulting aerosol and the desired spray flow rate. The derivatized carbohydrate surfactants which are used in the highly aqueous liquid carriers of the invention are C8-glucose, C9-glucose, C10-glucose, C12-glucose, and. C14-maltose and are described further in Table 1.

**Table 1.**

| Carbohydrate Surfactants | | |
|---|---|---|
| **Surfactant** | **Name** | **Molecular weight** |
| C8 - glucose* | n-Octyl-β-D-glucopyranoside | 292.4 |
| C9-glucose** | n-Nonyl-β-D-glucopyranoside | 308.4 |
| C10-glucose* | Decyl-β-D-glucopyranoside | 320.4 |
| C12 -glucosa** | n-Dodecyl-βB-D-glucopyranoside | 348.5 |
| C14 - maltose** | n-Tetradecyl-β-D-maltopyranoside | 538.6 |
| *Available from Sigma-Aldrich (www.sigma-aldrich, com) | ** Available from Anatrace (www.anatrace.com) | |

In general, the derivatized carbohydrate surfactants useful in the highly aqueous liquid formulations of the invention should be selected so that the surfactant is soluble in the carrier liquid. However, the derivatized carbohydrate may be suspended in the carrier liquid and still produce the desired surface tension of from about 20 dyne/cm to about 40 dyne/cm.

The derivatized carbohydrate surfactants described herein are capable of effectively reducing the surface tension of the liquid carrier vehicle to a range of from about 20 dyne/cm to from about 40 dyne/cm and preferably from about 25 dyne/cm to about 38 dyne/cm and more preferably from about 25 dyne/cm to about 30 dyne/cm.

The term "aerosol" as used herein refers to a suspension of fine particles (liquid or solid) in air with a range of particle sizes. (See Albert et.al., Comprehensive Respiratory Medicine, 1999, Mosby, London, pp. 7.36.1)

The term "MMD" stands for "Mass Median Diameter" of the aerosol and is the diameter about which 50% of the total particle mass resides.

The particle size of the aerosol droplets produced when the liquid carrier described herein is sprayed with an EHD device will range from about 1 µm to about 50 µm in diameter with the particular size of the aerosol droplet being selected depending on where in the respiratory tract the drug is to be delivered. Generally, if the drug is to be delivered to the deep lung for systemic activity, the particle size of the resulting aerosol will range from about 1 µm to about 8.0 µm and preferably from about 1 µm to about 5.0 µm. If the drug is to be delivered to the mid-lung, the particle size of the resulting aerosol will range from about 2 µm to about 10 µm and preferably from about 5 µm to about 10 µm will be used. If the pharmaceutically active agent is delivered to the oropharangeal region the particle size of the aerosol will generally range from about 2 µm to about 10 µm with a range of from about 5 µm to about 10 µm being preferred. If the drug is to be delivered to the buccal mulosar or to the nares, the particle size of the resulting aerosol will range from about 10 µm to about 50 µm and preferably from about 20 µm to about 30 µm will be used.

The term "resistivity" refers to the electrical resistance of a material, e.g., the liquid carrier per unit length, area, or volume. While the prior art suggests that a broad resistivity range may be used with EHD spraying devices, i.e., from 10² to 10⁸ Ohm meters, the base carrier liquids being sprayed were non-aqueous or slightly aqueous. The prior art generally suggests that relativity high resistivities of at least 10⁴ Ohm meters should be used to spray highly aqueous liquids.

Various liquid carrier vehicles were prepared as illustrated by the data shown in Tables 3-4; the following abbreviations are used in the Tables.

**Table 2.**

| Abbreviations Used in Tables 3-5 | | | |
|---|---|---|---|
| **Abbreviation** | **Component** | **Abbreviation** | **Component** |
| S1 | C8-Glucose | S5 | C14-Maltose |
| S2 | C9-Glucose | E1 | Vitamin E TPGS |
| S3 | C10-Glucose | E2 | PVP |
| S4 | C12-Glucose | | |

**Table 3.**

| 70% H₂O/30% ETOH Base Formulation | | | | | | |
|---|---|---|---|---|---|---|
| **Surfactant** | **Excipients** | **Resistivity** | **Flow** | **MMD** | **GSD** | **FPF₄.₇₉** |
| | **And/or** | **(Ω•m)** | **Rate** | **(µm)** | | **(%)** |
| | **Co-Solvent** | | **(µL/sec)** | | | |
| 0.5% S1 | 10% PEG | 242 | 10 | 1.75 | 1.90 | 92.66 |
| 0.5% S1 | 0.5% E2 + 10% PG | 202 | 8 | 1.89 | 1.55 | 99.99 |
| 0.5% S2 | - | 214 | 9 | 1.95 | 1.75 | 95.42 |
| 0.5% S2 | 0.1% E1 + 10% PG | 200 | 8 | 2.01 | 1.60 | 97.45 |
| 0.5% S3 | 10% PEG | 186 | 5 | 1.42 | 1.61 | 97.55 |
| 0.5% S3 | 0.1% E1 +,10% PG | 214 | 6 | 1.74 | 1.58 | 99.67 |
| 0.1% S4 | 10% PEG | 493 | 9 | 2.32 | 1.63 | 91.60 |
| 0.1% S4 | 0.1% E1 + 10% PG | 490 | 9 | 2.43 | 1.51 | 95.18 |
| 0.5% S5 | 0.1% E1 + 10% PG | 521 | 8 | 2.32 | 1.52 | 96.79 |
| 0.5% S5 | 0.5% E2 + 10% PG | 206 | 8 | 2.14 | 1.56 | 97.77 |

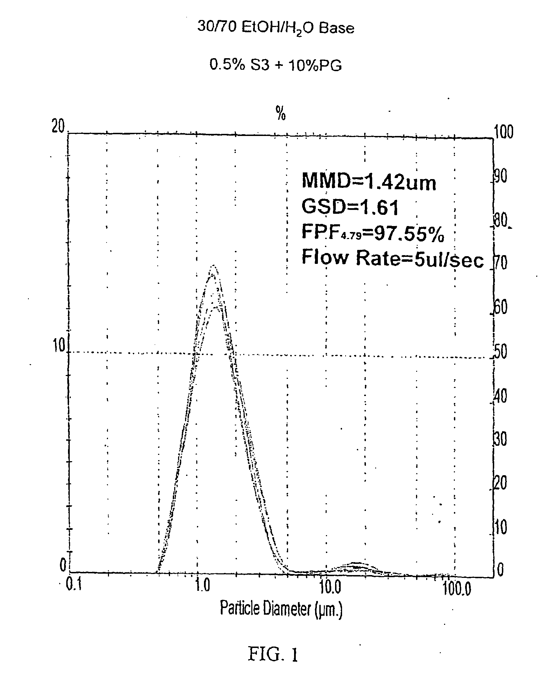

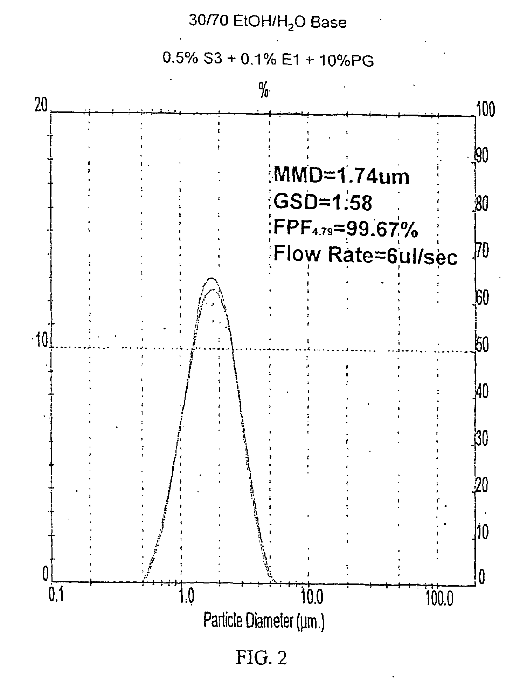

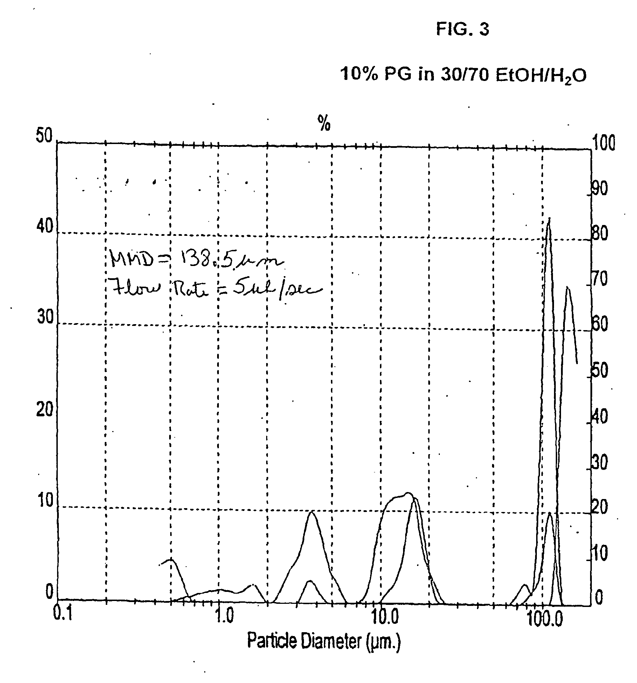

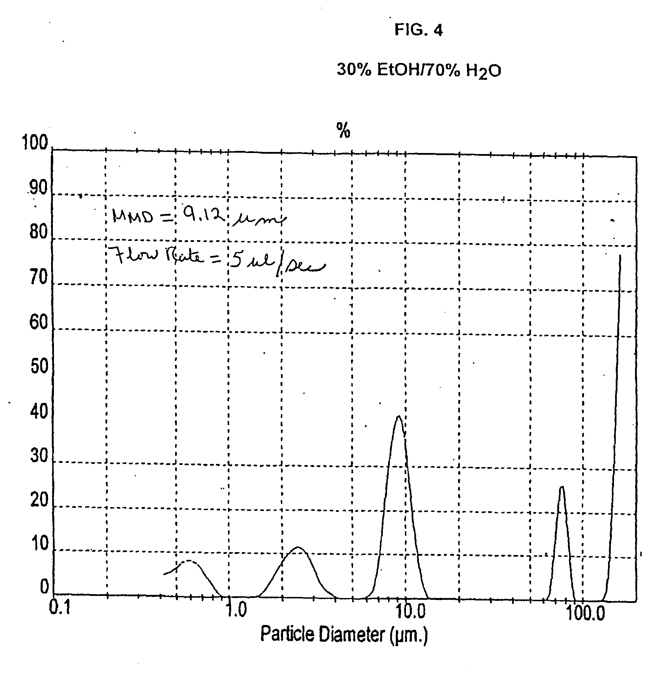

As illustrated by the data in Table 3 and the charts in FIG. 1 and FIG. 2, aerosols produced from the liquid carrier vehicles of the invention are nearly monodisperse in nature. As shown in FIG. 3 and FIG. 4, aerosols produced using 30/70 EtOH/H₂O and 30/70 EtOH/H₂O, plus 10% propylene glycol were polydisperse with MMD's of 9.12 µm and 138.5 µm respectively.

**Table 4.**

| 80% H₂O/20% ETOH Base Formulation | | | | | | |
|---|---|---|---|---|---|---|
| **Surfactant** | **Excipient** | **Resistivity** | **Flow** | **MMD** | **GSD** | **FPF_{4.79}** |
| | **And/or** | **(Ω•m)** | **Rate** | **(µm)** | | **(%)** |
| | **Co-Solvent** | | **(µL/sec)** | | | |
| 0.5%S2 | 0.1% E1 + 10% PG | 506 | 7 | 2.20 | 1.58 | 96.15 |
| 0.5% S2 | 0.1%E1+10%PG | 204 | 10 | 2.09 | 1.66 | 95.81 |
| 0.5%S2 | 0.5% E2 + 10% PG | 201 | 8 | 1.91 | 1.69 | 94.92 |
| 0.3% S2 | 0.5% E2 + 10% PG | 455 | 7 | 2.30 | 1.56 | 96.10 |
| 0.3% S2 | 0.5% E2 + 10% PG | 206 | 7 | 2.62 | 1.56 | 91.76 |
| 0.5% S3 | 0.5% E2+ 10% PG | 200 | 5 | 1.68 | 1.27 | 100.0 |
| 0.3% S3 | 10% PEG | 205 | 5 | 1.45 | 1.39 | 100.0 |
| 0.3% S3 | 10% PEG | 205 | 10 | 2.09 | 1.77 | 93.34 |
| 0.3% S3 | 10% PEG | 219 | 7 | 2.89 | 1.56 | 87.47 |
| 0.5% S4 | 10% PEG | 218 | 7 | 2.41 | 1.97 | 84.27 |

The liquid carrier vehicles of the invention may be sprayed at relatively fast flow rates, i.e., on the order of 5 to 10 µl/sec, as opposed to µl/min taught by Gomez. A faster flow rate is important to a patient being treated as a faster flow rate translates into less time it takes for the patient to be treated.

**Table 5.**

| 100% H₂O Base Formulation | | | | | | |
|---|---|---|---|---|---|---|
| **Surfactant** | **Excipient** | **Resistivity** | **Flow** | **MMD** | **GSD** | **FPF_{4.79}** |
| | **And/Or** | **(Ω•m)** | **Rate** | **(µm)** | | **(%)** |
| | **Co-Solvent** | | **(µL/sec)** | | | |
| 1% S1 | 1% E2 + 10% PG | 199 | 7 | 3.26 | 1.62 | 78.32 |
| 1% 51 | 1 % E2 + 10% PG | 199 | 10 | 4.25 | 1.44 | 62.30 |
| 1%S2 | 1% E2 + 10% PG | 197 | 7 | 2.18 | 1.82 | 91.87 |
| 1% S2 | 1% E2+10% PG | 201 | 7 | 2.87 | 1.76 | 81.23 |
| 1% S2 | 1% E2 + 10% PG | 201 | 12 | 3.18 | 1.75 | 76.33 |
| 0.3% S3 | 10% PG | 524 | 5 | 3.21 | 1.43 | 86.86 |

A comparison of the data presented in Tables 4-5 indicate that as the amount of water in the liquid carrier vehicle of the invention increases, the MMD of the aerosol slowly increases. At the same time, the GSD remains about the same evidencing the nearly monodisperse character of the aerosols of the invention.

## Claims

1. A liquid carrier vehicle for a dissolved or suspended pharmaceutically active agent administered to a patient via inhalation of an aerosol wherein said aerosol is produced by an electrohydrodynamic spraying device, said liquid carrier vehicle consists of:
a. from 50% V/V to 100% V/V water;
b. from 0% to 40%V/V ethanol;
c. from 0.5% to 30% V/V of a co-solvent
d. from 0.5 % to 10% W/V of a pharmaceutical acceptable excipient selected from the group consisting of antioxidants, antimicrobials, pH adjusting acids and bases, tonicity adjusting agents and viscosity adjusting agents; and
e. from 0.05% W/V to 10% W/V of a derivatized carbohydrate surfactant selected from the group consisting of n-octyl-β-D-glucopyranoside, n-nonyl-β-D-glucopyranoside, decyl-p-D-glucopyranoside, n-dodecyl-β-D-glucopyranoside, and n-tetradecyl-β-D-maltopyranoside;
wherein said liquid carrier has a resistivity of from 25 ohm m to 8000 ohm m and a surface tension of from 20•10⁻³ N/m (20 dyne/cm) to 40•10⁻³ N/m (40 dyne/cm).

2. The liquid carrier vehicle according to claim 1 wherein said carrier vehicle consists of :
a. from 70% V/V to 100% V/V water;
b. from 0% to 30% V/V ethanol;
c. from 2.5% to 10% V/V of a co-solvent
d. from 0.5% to 5% W/V of said pharmaceutically acceptable excipient; and
e. from 0.3% W/V to 5% W/V of said derivatized carbohydrate surfactant;
wherein said liquid carrier has a resistivity of from 100 ohm m to 500 ohm m and a surface tension of from 25•10⁻³ N/m (25 dyne/cin) to 30•10⁻³ N/m (30 dyne/cm).

3. The liquid carrier vehicle according to claim 2 wherein said carrier vehicle consist of:
a. from 80% V/V to 100% V/V waster;
b. from 0% to 20% V/V ethanol;
c. from 5% to 10% V/V of a co-solvent ;
d. from 0.5% to 5% W/V of said pharmaceutical acceptable excipient; and
e. from 0.5% W/V to 1 % W/V of said derivatized carbohydrate surfactant;
wherein said liquid carrier has a resistivity of from 100 ohm m to 500 ohm m add a surface tension of from 25•10⁻³ N/m (25 dyne/cm) to 30•10⁻³ N/m (30 dyne/cm).

4. The liquid carrier vehicle according to claim 1 wherein said carrier vehicle contains from 70% V/V to 100% V/V water.

5. The liquid carrier vehicle according to claim 1 wherein said pharmaceutically acceptable excipient is present in said liquid carrier vehicle at from 0.5% WN to 5% W/V.

6. The liquid carrier vehicle according to claim 5 wherein said pharmaceutically acceptable excipient is 0.5% WN polyvinyl pyrrolidone.

7. The liquid carrier vehicle according to claim 1 wherein said co-solvent is present in said liquid carrier vehicle at from 2.5%V/V to 10% V/V.

8. The liquid carrier vehicle according to claim 7 wherein said co-solvent is present in said liquid carrier vehicle at from 2.5% V/V to 5% V/V.

9. The liquid carrier vehicle according to claim 1 wherein said co-solvent is selected form the group consisting of propylene glycol, glycerol, and polyethylene glycol.

10. The liquid carrier vehicle according to claim 9 wherein said co-solvent is 5% VN propylene glycol.

11. The liquid carrier vehicle according to claim 1 wherein said liquid carrier has a resistivity of from 100 ohm m to 5,00 ohm m and a surface tension of from 20•10⁻³ N/m (20 dyne/cm) to 30•10⁻³ N/m (30 dyne/cm).

12. The liquid carrier vehicle according to claim 1 wherein said surfactant is present in said liquid carrier vehicle at 0.3% WN to from 5% W/V.

13. The liquid carrier vehicle according to any of claims 1 to 12 further containing an effective amount of an active agent.

14. Use of the liquid carrier vehicle according to claim 13 for the preparation of a medicament in the form of an aerosol to be administered to the pulmonary tract of a patient using an electrohydrodynamic spraying/aerosolization means.

## Patentansprüche

1. Flüssiges Trägerverhikel für einen gelösten oder suspendierten pharmazeutischen Wirkstoff, der einem Patienten über Inhalation eines Aerosols verabreicht wird, wobei das Aerosol durch eine elektrohydrodynamische Sprühvorrichtung erzeugt wird, wobei das flüssige Trägervehikel besteht aus:
a. 50% (Vol./Vol.) bis 100% (Vol./Vol.) Wasser;
b. 0% bis 40% (Vol./Vol.) Ethanol;
c. 0% bis 30% (Vol./Vol.) eines Cosolvens;
d. 0,5% bis 10% (Gew./Vol.) eines pharmazeutisch verträglichen Hilfsstoffs, ausgewählt aus der Gruppe bestehend aus Antioxidantien, antimikrobiellen Mitteln, den pH einstellenden Säuren und Basen, die Tonizität einstellenden Mitteln und die Viskosität einstellenden Mitteln; und
e. 0,05% (Gew./Vol.) bis 10% (Gew./Vol.) eines derivatisierten Kohlenhydrat-Tensids, das ausgewählt ist aus der Gruppe bestehend aus n-Octyl-β-D-glucopyranosid, n-Nonyl-β-D-glucopyranosid, Decyl-p-D-glucopyranosid, n-Dodecyl-β-D-glucopyranosid und n-Tetradecyl-β-D-maltopyranosid;
wobei der flüssige Träger einen spezifischen Widerstand von 25 Ohm·m bis 8000 Ohm·m und eine Oberflächenspannung von 20·10⁻³ N/m (20 Dyn/cm) bis 40·10⁻³ N/m (40 Dyn/cm) aufweist.

2. Flüssiges Trägervehikel nach Anspruch 1, wobei das Trägervehikel besteht aus:
a. 70% (Vol./Vol.) bis 100% (Vol./Vol.) Wasser;
b. 0% bis 30% (Vol./Vol.) Ethanol;
c. 2,5% bis 10% (Vol./Vol.) eines Cosolvens;
d. 0,5% bis 5% (Gew./Vol.) des pharmazeutisch verträglichen Hilfsstoffs; und
e. 0,3% (Gew./Vol.) bis 5% (Gew./Vol.) des derivatisierten Kohlenhydrat-Tensids;
wobei der flüssige Träger einen spezifischen Widerstand von 100 Ohm·m bis 500 Ohm·m und eine Oberflächenspannung von 25·10⁻³ N/m (25 Dyn/cm) bis 30·10⁻³ N/m (30 Dyn/cm) aufweist.

3. Flüssiges Trägervehikel nach Anspruch 2, wobei das Trägervehikel besteht aus:
a. 80% (Vol./Vol.) bis 100% (Vol./Vol.) Wasser;
b. 0% bis 20% (Vol./Vol.) Ethanol;
c. 5% bis 10% (Vol./Vol.) eines Cosolvens;
d. 0,5% bis 5% (Gew./Vol.) des pharmazeutisch verträglichen Hilfsstoffs; und
e. 0,5% (Gew./Vol.) bis 1 % (Gew./Vol.) des derivatisierten Kohlenhydrat-Tensids;
wobei der flüssige Träger einen spezifischen Widerstand von 100 Ohm·m bis 500 Ohm·m und eine Oberflächenspannung von 25·10⁻³ N/m (25 Dyn/cm) bis 30·10⁻³ N/m (30 Dyn/cm) aufweist.

4. Flüssiges Trägervehikel nach Anspruch 1, wobei das Trägervehikel 70% (Vol./Vol.) bis 100% (Vol./Vol.) Wasser enthält.

5. Flüssiges Trägervehikel nach Anspruch 1, wobei der pharmazeutisch verträgliche Hilfsstoff in dem flüssigen Trägervehikel zu 0,5% (Gew.Nol.) bis 5% (Gew.Nol.) vorliegt.

6. Flüssiges Trägervehikel nach Anspruch 5, wobei der pharmazeutisch verträgliche Hilfsstoff 0,5% (Gew./Vol.) Polyvinylpyrrolidon ist.

7. Flüssiges Trägervehikel nach Anspruch 1, wobei das Cosolvens in dem flüssigen Trägervehikel zu 2,5% (Vol./Vol.) bis 10% (Vol./Vol.) vorliegt.

8. Flüssiges Trägervehikel nach Anspruch 7, wobei das Cosolvens in dem flüssigen Trägervehikel zu 2,5% (Vol./Vol.) bis 5% (Vol./Vol.) vorliegt.

9. Flüssiges Trägervehikel nach Anspruch 1, wobei das Cosolvens aus der Gruppe bestehend aus Propylenglycol, Glycerol und Polyethylenglycol ausgewählt ist.

10. Flüssiges Trägervehikel nach Anspruch 9, wobei das Cosolvens 5% (Vol./Vol.) Propylenglycol ist.

11. Flüssiges Trägervehikel nach Anspruch 1, wobei der flüssige Träger einen spezifischen Widerstand von 100 Ohm·m bis 500 Ohm·m und eine Oberflächenspannung von 20·10⁻³ N/m (20 Dyn/cm) bis 30·10⁻³ N/m (30 Dyn/cm) aufweist.

12. Flüssiges Trägervehikel nach Anspruch 1, wobei das Tensid in dem flüssigen Trägervehikel zu 0,3% (Gew./Vol.) bis 5% (Gew./Vol.) vorliegt.

13. Flüssiges Trägervehikel nach einem der Ansprüche 1 bis 12, welches ferner eine wirksame Menge eines Wirkstoffs enthält.

14. Verwendung des flüssigen Trägervehikels nach Anspruch 13 für die Herstellung eines Arzneimittels in Form eines Aerosols, das dem Lungentrakt eines Patienten unter Verwendung eines elektrohydrodynamischen Sprüh-/Vernebelungsmittels zu verabreichen ist.

## Revendications

1. Substance liquide de support destiné à un agent pharmaceutiquement actif dissous ou suspendu administré à un patient par inhalation d'un aérosol, dans laquelle ledit aérosol est produit par un dispositif de pulvérisation électrohydrodynamique, ladite substance liquide de support étant constituée de :
a. 50 % en volume à 100 % en volume d'eau ;
b. 0 % à 40 % en volume d'éthanol ;
c. 0 % à 30 % en volume d'un cosolvant ;
d. 0,5 % à 10 % en poids/volume d'un excipient pharmaceutiquement acceptable choisi dans le groupe constitué d'antioxydants, d'anti-microbiens, d'acides et de bases permettant d'ajuster le pH, d'agents permettant d'ajuster la tonicité et d'agents permettant d'ajuster la viscosité ; et
e. 0,05 % en poids/volume à 10 % en poids/volume d'un tensioactif à base d'un dérivé de glucide choisi dans le groupe constitué par le n-octyl-β-D-gluco-pyranoside, le n-nonyl-β-D-glucopyranoside, le décyl-p-D-glucopyranoside, le n-dodécyl-β-D-glucopyranoside et le n-tétradédyl-β-D-maltopyranoside ;
dans lequel ledit support liquide possède une résistivité de 25 ohm m à 8000 ohm m et une tension superficielle de 20·10⁻³ N/m (20 dyne/cm) à 40.10⁻³ N/m (40 dyne/cm) .

2. Substance liquide de support selon la revendication 1, dans laquelle ladite substance de support est constituée de :
a. 70 % en volume à 100 % en volume d'eau ;
b. 0 % à 30 % en volume d'éthanol ;
c. 2,5 % à 10 % en volume d'un cosolvant ;
d. 0,5 % à 5 % en poids/volume dudit excipient pharmaceutiquement acceptable ; et
e. 0,3 % en poids/volume à 5 % en poids/volume dudit tensioactif à base d'un dérivé de glucide ;
dans lequel ledit support liquide possède une résistivité de 100 ohm m à 500 ohm m et une tension superficielle de 25.10⁻³ N/m (25 dyne/cm) à 30.10⁻³ N/m (30 dyne/cm).

3. Substance liquide de support selon la revendication 2, dans laquelle ladite substance de support est constituée de :
a. 80 % en volume à 100 % en volume d'eau ;
b. 0 % à 20 % en volume d'éthanol ;
c. 5 % à 10 % en volume d'un cosolvant ;
d. 0,5 % à 5 % en poids/volume dudit excipient pharmaceutiquement acceptable ; et
e. 0,5 % en poids/volume à 1 % en poids/volume dudit tensioactif à base d'un dérivé de glucide ;
dans lequel ledit support liquide possède une résistivité de 100 ohm m à 500 ohm m et une tension superficielle de 25.10⁻³ N/m (25 dyne/cm) à 30.10⁻³ N/m. (30 dyne/cm).

4. Substance liquide de support selon la revendication 1, dans laquelle ladite substance de support contient 70 % en volume à 100 % en volume d'eau.

5. Substance liquide de support selon la revendication 1, dans laquelle 0,5 % en poids/volume à 5 % en poids/volume dudit excipient pharmaceutiquement acceptable sont présents dans ladite substance liquide de support.

6. Substance liquide de support selon la revendication 5, dans laquelle ledit excipient pharmaceutiquement acceptable est la polyvinyl-pyrrolidone à 0,5 % en poids/volume.

7. Substance liquide de support selon la revendication 1, dans laquelle 2,5 % en volume à 10 % en volume dudit cosolvant sont présents dans ladite substance liquide de support.

8. Substance liquide de support selon la revendication 7, dans laquelle 2,5 % en volume à 5 % en volume dudit cosolvant sont présents dans ladite substance liquide de support.

9. Substance liquide de support selon la revendication 1, dans laquelle ledit cosolvant est choisi dans le groupe constitué par le propylène glycol, le glycérol et le polyéthylène glycol.

10. Substance liquide de support selon la revendication 9, dans laquelle ledit cosolvant est le propylène glycol à 5 % en volume.

11. Substance liquide de support selon la revendication 1, dans laquelle ledit support liquide possède une résistivité de 100 ohm m à 500 ohm m et une tension superficielle de 20.10⁻³ N/m (20 dyne/cm) à 30.10⁻³ N/m (30 dyne/cm) .

12. Substance liquide de support selon la revendication 1, dans laquelle 0,3 % en poids/volume à 5 % en poids/volume dudit tensioactif sont présents dans ladite substance liquide de support.

13. Substance liquide de support selon l'une quelconque des revendications 1 à 12, contenant en outre une quantité efficace d'agent actif.

14. Utilisation de la substance liquide de support selon la revendication 13 pour la préparation d'un médicament sous la forme d'un aérosol à administrer par voie pulmonaire à un patient en utilisant un moyen électrohydrodynamique de pulvérisation/aérosolisation.
